# FASCICULE DE BREVET EUROPEEN

(11) **EP 2 417 092 B1**
(45) Date de publication et mention de la délivrance du brevet: **06.01.2016**
(21) Numéro de dépôt: 10711422.5
(22) Date de dépôt: 30.03.2010
(51) Int. Cl.: C07C 37/60, C07C 39/08

(54) **PROCEDE D'HYDROXYLATION DE PHENOLS ET D'ETHERS DE PHENOLS**
VERFAHREN ZUR HYDROXYLIERUNG VON PHENOLEN UND PHENOLETHERN
PROCESS FOR THE HYDROXYLATION OF PHENOLS AND OF PHENOL ETHERS

(30) Priorité: 06.04.2009 FR 0901660
(43) Date de publication de la demande: 15.02.2012
(73) Titulaire: Rhodia Opérations, 75009 Paris (FR)
(72) Inventeur: GAREL, Laurent, F-69003 Lyon (FR)
(74) Mandataire: Menville, Laure
(86) Numéro de dépôt international: PCT/EP2010/054232
(87) Numéro de publication internationale: WO 2010/115784

(56) Documents cités:
- FR-A- 2 071 464
- FR-A- 2 318 851
- FR-A- 2 655 332

## Description

La présente invention a pour objet un procédé d'hydroxylation de phénols et d'éthers de phénols par le peroxyde d'hydrogène.

L'invention vise plus particulièrement un procédé d'hydroxylation du phénol par le peroxyde d'hydrogène.

La réaction d'hydroxylation du phénol n'est pas sélective et l'on obtient deux isomères à savoir le 1,4-dihydroxybenzène ou hydroquinone (HQ) et le 1,2-dihydroxybenzène ou pyrocatéchol (PC).

La proportion des deux diphénols peut varier assez largement et dépend du procédé utilisé.

La production des diphénols à l'échelle industrielle nécessite des installations dédiées à un procédé donné qui peut conduire de manière prépondérante soit à l'hydroquinone, soit au pyrocatéchol.

A l'heure actuelle, le marché étant demandeur de davantage de pyrocatéchol, on est donc à la recherche d'un procédé d'hydroxylation du phénol qui conduise de manière prépondérante au pyrocatéchol.

Il s'avère donc que pour répondre à la demande du marché, il est important, de disposer d'un procédé industriel permettant d'augmenter la production de pyrocatéchol formé par rapport à la quantité d'hydroquinone.

De nombreux procédés d'hydroxylation des phénols sont décrits dans l'état de la technique.

Citons, entre autres, le brevet FR-A 2 071 464 qui concerne un procédé industriel très important d'hydroxylation de phénols et d'éthers de phénols qui permet d'accéder en particulier à l'hydroquinone et au pyrocatéchol lors de l'application de ce procédé au phénol.

Ledit procédé consiste à réaliser l'hydroxylation, par le peroxyde d'hydrogène, en présence d'un acide fort. Parmi ces acides forts, l'acide sulfurique, l'acide p-toluènesulfonique, l'acide perchlorique sont les plus utilisés.

Bien que ce procédé soit très intéressant, il présente l'inconvénient de nécessiter à basse température, la mise en oeuvre d'une quantité importante de catalyseur allant jusqu'à 20 % du poids de peroxyde d'hydrogène mis en oeuvre. Dans le cas d'une quantité moindre, une durée de réaction plus importante est requise, par exemple 10 heures.

L'hydroxylation du phénol est décrite dans FR-A 2 318 851, au sein de l'acide trifluorométhanesulfonique et dans FR-A 2 655 332, en présence d'une quantité efficace d'au moins un sel de métal alcalin ou alcalino-terreux d'au moins un acide protonique ayant un pKa dans l'eau inférieur à - 0,1 et d'une quantité efficace de l'acide protonique libre.

Dans les trois brevets précités, il est décrit la mise en oeuvre d'un oxacide du phosphore comme par exemple l'acide phosphorique.

Ce dernier n'est pas cité comme acide protonique fort mais comme agent complexant des ions métalliques (Fe, Cu, Cr, Co, Mn, V) apportés par les réactifs et qui ont la particularité de dégrader le rendement réactionnel en produits d'hydroxylation.

Par ailleurs, on connaît selon FR-A 2 266 683, un procédé qui consiste à effectuer l'hydroxylation du phénol, en présence d'une cétone. Il en résulte une amélioration du rendement de la réaction en hydroquinone et pyrocatéchol. Tous les exemples décrits conduisent à une quantité de pyrocatéchol plus grande que celle d'hydroquinone et le ratio PC/HQ varie seulement entre 1 et 1,72.

Dans EP-A 0 480 800, on a proposé au contraire, un procédé permettant d'accroître la quantité d'hydroquinone formée par rapport à la quantité de pyrocatéchol, en mettant en oeuvre une cétone de type aromatique.

Conformément au procédé décrit dans EP- A 0 480 800, la présence de ce type de cétone lors de l'hydroxylation du phénol joue sur la régiosélectivité de la réaction et des rapports PC/HQ variant entre 0,9 et 1,1 sont avantageusement obtenus.

L'un des objectifs de l'invention est de fournir un procédé d'hydroxylation du phénol permettant d'accroître la quantité de pyrocatéchol formé par rapport à la quantité d'hydroquinone.

Un autre objectif de l'invention est de fournir un procédé d'hydroxylation du phénol qui permette d'obtenir plus de pyrocatéchol tout en conservant des rendements en diphénols élevés.

Par ailleurs, le problème est qu'au cours du temps, la demande du marché est fluctuante et requiert des quantités variables de pyrocatéchol et d'hydroquinone.

Il est difficile d'adapter la production des diphénols des unités industrielles à la demande du marché.

Un autre objectif de l'invention est donc de fournir un procédé souple permettant de moduler ainsi le ratio pyrocatéchol/hydroquinone et ainsi d'adapter aisément la production à la demande du marché.

Plus particulièrement, la présente invention a pour objet un procédé d'hydroxylation d'un phénol ou d'un éther de phénol présentant au moins un atome d'hydrogène en position ortho et para du groupe hydroxyle ou éther, par réaction dudit phénol ou éther de phénol, avec le peroxyde d'hydrogène, en présence d'un catalyseur acide, caractérisé par le fait que la réaction est conduite en présence d'une quantité efficace d'un catalyseur qui est un mélange d'au moins deux acides forts et qui est caractérisé par le fait que l'on met en oeuvre un mélange d'au moins deux acides :
- l'un des acides étant choisi parmi les acides protoniques forts présentant un pKₐ (S) supérieur ou égal à celui de l'acide sulfurique et un ΔpKₐ (S) par rapport à l'acide sulfurique inférieur ou égal à 4 et supérieur ou égal à 0,
- et l'autre acide étant choisi parmi les superacides.

Dans le présent exposé de l'invention, on désigne par « acide protonique fort », un acide présentant un pKₐ (S) supérieur ou égal à celui de l'acide sulfurique : (S) représentant le solvant organique qui est le nitrobenzène.

On entend par « superacide », un acide présentant un pKₐ (S) inférieur à celui de l'acide sulfurique.

Le pKₐ (S) est défini comme la constante de dissociation ionique du couple acide/base dans un solvant (S).

On définit le pKₐ des acides convenant au procédé de l'invention, par référence à une mesure de potentiométrie effectuée dans un solvant qui est le nitrobenzène (S) et dont le protocole de mesure est exposé avant les exemples.

Les acides intervenant dans le procédé de l'invention sont définis plus loin, par une différence de pKₐ, Δ pKₐ qui correspond pour un même solvant, à la différence entre le pKₐ de l'acide choisi et le pKₐ de l'acide sulfurique.

On a constaté de manière inattendue, que la mise en oeuvre lors de l'hydroxylation du phénol par le peroxyde d'hydrogène, d'un mélange d'acides présentant une acidité différente, exerce une action sur la régiosélectivité de la réaction et permet selon le choix des acides d'augmenter la formation du pyrocatéchol, en augmentant la production de ce composé par rapport à l'hydroquinone.

On a également trouvé que l'on pouvait moduler le ratio pyrocatéchol/hydroquinone obtenu en faisant varier les proportions relatives des différents acides du mélange.

Il est à noter que les oxacides du phosphore décrits dans la littérature définis comme étant des composés à fonction acide du phosphore au degré d'oxydation + 5, ne sont pas considérés comme des acides protoniques forts au sens de l'invention. Un exemple comparatif fournit dans la partie des exemples met en évidence qu'un acide phosphorique ne peut pas être considéré comme un équivalent de l'acide sulfurique ou autre acide protonique fort de l'invention.

Selon une variante du procédé de l'invention, les oxacides du phosphore peuvent être mis en oeuvre dans le procédé de l'invention pour remplir leur fonction d'agents complexants, en plus du mélange d'acides préconisés par l'invention.

Le procédé de l'invention s'applique aussi bien aux phénols qu'aux éthers de phénols et qui sont désignés dans le présent texte, par le terme « substrat ».

Le procédé de l'invention est bien adapté à l'hydroxylation du phénol mais également aux composés phénoliques substitués.

Par « composés phénoliques substitués », on entend un composé aromatique dont l'un des atomes d'hydrogène du cycle aromatique est remplacé par un groupe hydroxyle et au moins un autre atome d'hydrogène, est remplacé par un substituant.

La nature du ou des substituants est indifférente dans la mesure où ils ne perturbent pas l'obtention du produit désiré.

La présente invention s'applique tout particulièrement aux substrats de formule générale (I) : 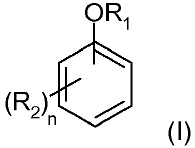 dans ladite formule :
- n est un nombre de 0 à 4, de préférence égal à 0, 1 ou 2,
- R₁ représente un atome d'hydrogène, un groupe alkyle, cycloalkyle, aryle, aralkyle,
- R₂, identiques ou différents, représentent un groupe alkyle, un groupe alkoxy, un groupe hydroxyle, un atome d'halogène, un groupe halogéno- ou perhalogénoalkyle.

Dans la formule (I), le groupe OR₁ est un groupe éther dès lors que R₁ est différent d'un atome d'hydrogène.

Dans le cadre de l'invention, on entend par « alkyle », une chaîne hydrocarbonée linéaire ou ramifiée en C₁-C₁₅, de préférence en C₁-C₁₀ et encore plus préférentiellement en C₁-C₄. Des exemples de groupes alkyle préférés sont notamment méthyle, éthyle, propyle, isopropyle, butyle, isobutyle, t-butyle.

Par « alkoxy », on entend un groupe alkyl-O- dans lequel le terme alkyle a la signification donnée ci-dessus. Des exemples préférés de groupes alkoxy sont les groupes méthoxy ou éthoxy.

Par « cycloalkyle », on entend un groupe hydrocarboné cyclique, monocyclique en C₃-C₈, de préférence, un groupe cyclopentyle ou cyclohexyle ou polycyclique (bi- ou tricyclique) en C₄-C₁₈, notamment adamantyle ou norbornyle.

Par « aryle », on entend un groupe mono- ou polycyclique aromatique, de préférence, mono- ou bicyclique en C₆-C₂₀, de préférence, phényle ou naphtyle. Lorsque le groupe est polycyclique c'est-à-dire qu'il comprend plus d'un noyau cyclique, les noyaux cycliques peuvent être condensés deux à deux ou rattachés deux à deux par des liaisons σ. Des exemples de groupes (C₆-C₁₈)aryle sont notamment phényle, naphtyle.

Par « aralkyle », on entend un groupe hydrocarboné, linéaire ou ramifié porteur d'un cycle aromatique monocyclique en C₇-C₁₂, de préférence, benzyle : la chaîne aliphatique comprenant 1 ou 2 atomes de carbone

Par « groupe halogénoalkyle », on entend un groupe alkyle tel que précédemment défini dont l'un ou plusieurs atomes d'hydrogène sont remplacés par un atome d'halogène, de préférence un atome de fluor.

Par « groupe perhalogénoalkyle », on entend un groupe alkyle comprenant de 1 à 10 atomes de carbone et de 3 à 21 atomes d'halogène de préférence de fluor et plus particulièrement le groupe trifluorométhyle.

Par « atome d'halogène », on définit le fluor, le chlore et le brome.

Le procédé de l'invention s'applique préférentiellement aux substrats répondant à la formule (I) dans laquelle n est égal à 0 ou 1 ; R₁ représente un atome d'hydrogène, un groupe alkyle ayant de 1 à 4 atomes de carbone ; R₂ représente un atome d'hydrogène, un groupe alkyle ou alkoxy ayant de 1 à 4 atomes de carbone.

Les substrats auxquels s'applique le procédé de l'invention sont notamment, le phénol ; les éthers aliphatiques de phénols ; les monoalkylphénols, les dialkylphénols, les trialkylphénols avec des groupes alkyle en C₁-C₄ ; les alkoxyphénols avec des groupes alkoxy en C₁-C₄.

Parmi les substrats de formule (I) qui pourront être mis en oeuvre dans le procédé de l'invention, on peut citer à titre non limitatif, le phénol ; les éthers aliphatiques de phénol tels que l'anisole, le phénétole ; les alkylphénols tels que l'o-crésol, le m-crésol ; les alkoxyphénols tels que le 2-méthoxyphénol (le gaïacol), le 2-éthoxyphénol (le guétol).

Le présent procédé convient tout particulièrement bien à la préparation d'hydroquinone et de pyrocatéchol à partir du phénol.

Conformément au procédé de l'invention, le choix de la mise en oeuvre d'un mélange d'acides et le choix des acides intervenant dans ledit mélange joue sur la régiosélectivité de la réaction.

La caractéristique du procédé de l'invention est de faire appel en tant que catalyseur de réaction, à un mélange d'au moins deux acides qui présentent une acidité différente.

Comme mentionné précédemment l'acide protonique fort, est un acide présentant un pKₐ (S) supérieur ou égal à celui de l'acide sulfurique.

Les acides protoniques forts mis en oeuvre dans le procédé de l'invention présentent un ΔpKₐ (S) par rapport à l'acide sulfurique inférieur ou égal à 4 et supérieur ou égal à 0.

Encore plus préférentiellement, les acides protoniques forts ont un ΔpKₐ (S) par rapport à l'acide sulfurique inférieur ou égal à 3 et supérieur ou égal à 0.

Comme exemples d'acides protoniques forts susceptibles d'être mis en oeuvre dans le procédé de l'invention, on peut citer notamment l'acide sulfurique, les acides sulfoniques aliphatiques ou aromatiques tels que par exemple, l'acide méthanesulfonique, l'acide éthanesulfonique, l'acide benzènesulfonique, les acides toluènesulfoniques, les acides naphtalènesulfoniques.

Une autre classe d'acides protoniques forts sont les acides hydroxybenzènesulfoniques, les acides hydroxybenzoïques sulfonés ; les acides hydroxybenzènedisulfoniques, les acides dihydroxybenzènedisulfoniques les acides hydroxytoluènesulfoniques, les acides hydroxynaphtalènesulfoniques et les acides hydroxynaphtalène-disulfoniques et leurs mélanges.

Parmi les acides hydroxybenzènesulfoniques, on utilisera de préférence l'acide 4-hydroxybenzènesulfonique, l'acide 2-hydroxybenzènesulfonique, l'acide 5-sulfosalicylique ou leur mélange.

Les acides dihydroxybenzènedisulfoniques préférés sont l'acide 5,6-dihydroxy-1,3-benzènedisulfonique, l'acide 4,6-dihydroxy-1,3-benzènedisulfonique, l'acide 2,5-dihydroxy-1,4-benzènedisulfonique.

Comme autres exemples d'acides, on peut citer notamment les acides perhalogénoacétiques tels que l'acide trichloroacétique, l'acide trifluoroacétique.

Pour ce qui est du deuxième composant du mélange d'acides, il s'agit d'un superacide c'est-à-dire un acide présentant un pKₐ (S) inférieur à celui de l'acide sulfurique et qui présente donc un ΔpKₐ négatif.

La borne inférieure n'est pas critique mais généralement le ΔpKₐ dans le nitrobenzène est supérieur ou égal à - 12.

Les superacides choisis préférentiellement dans le procédé de l'invention présentent un ΔpKₐ inférieur ou égal à - 0,1 et de préférence supérieur ou égal à - 8.

Parmi les superacides convenant au procédé de l'invention, on peut citer plus particulièrement, l'acide perchlorique, les acides halogénosulfoniques tels que l'acide fluorosulfonique, l'acide chlorosulfonique ; les acides perhalogénoalcanesulfoniques, de préférence l'acide trifluorométhane-sulfonique.

Egalement comme superacides, on peut mentionner entre autres, l'acide trifluorométhanesulfinique ; l'acide bis-trifluorométhanesulfonimide.

Conformément au procédé de l'invention, on fait appel à une catalyse mixte c'est-à-dire que l'on met en oeuvre deux acides.

On ne sortira pas du cadre de l'invention, à ajouter un acide supplémentaire. Ainsi, l'invention inclut le cas où l'on peut met en oeuvre un mélange d'acides protoniques forts ou un mélange de superacides.

Comme couples d'acides choisis préférentiellement, on peut mentionner l'acide perchlorique et l'acide sulfurique ; l'acide perchlorique et l'acide 4-hydroxybenzènesulfonique ; l'acide trifluorométhanesulfonique et l'acide 4-hydroxybenzènesulfonique ; l'acide bis-trifluorométhanesulfonimide et l'acide 4-hydroxybenzènesulfonique.

La mise en oeuvre d'une catalyse mixte telle précitée par rapport à une catalyse simple à l'aide d'un seul acide protonique fort permet d'accroître la quantité de pyrocatéchol formé par rapport à la quantité d'hydroquinone.

Le procédé de l'invention permet d'obtenir des ratios pyrocatéchol/hydroquinone pouvant varier avantageusement entre 1,4 et 2,2.

La proportion des différents acides peut varier largement.

Ainsi, on peut mettre en oeuvre des mélanges comprenant :
- de 60 % à 95 % en moles d'un acide protonique fort,
- de 5 % à 40 % en moles d'un superacide.

Les mélanges mis en oeuvre préférentiellement comprennent :
- de 80 % à 95 % en moles d'un acide protonique fort,
- de 5 % à 20 % en moles d'un superacide.

Chaque pourcentage d'acide exprime le rapport (exprimé en %) entre le nombre de moles de l'acide considéré et le nombre de moles de la somme des deux acides (acide protonique fort + superacide).

Par rapport à un mélange d'acides définis, la variation de la proportion relative de chaque acide permet de faire varier le ratio pyrocatéchol/hydroquinone obtenu.

Ainsi, le procédé de l'invention permet d'obtenir un ratio modulable qui permet ainsi d'adapter la production à la demande du marché.

Les acides mis en oeuvre dans le mélange sont disponibles dans le commerce sous forme solide, liquide ou en solution aqueuse dont la concentration peut varier entre 5 et 95 % en poids, de préférence entre 50 et 70 % en poids.

La quantité d'acide protonique fort mise en oeuvre exprimée par le rapport entre le nombre de moles dudit acide et le nombre de moles de substrat varie avantageusement entre 0,01 % et 0,1 %. Ainsi, ledit rapport molaire est choisi de préférence entre 0,015 % et 0,06 %.

La quantité de superacide mise en oeuvre exprimée par le rapport entre le nombre de moles dudit acide et le nombre de moles de substrat varie avantageusement entre 0,002 % et 0,05 %. Ainsi, ledit rapport molaire est choisi de préférence entre 0,003 % et 0,03 %.

Conformément au procédé de l'invention, on fait réagir un phénol ou un éther de phénol avec le peroxyde d'hydrogène, en présence du mélange d'acides tels que définis.

Le peroxyde d'hydrogène mis en oeuvre selon l'invention peut être sous forme de solution aqueuse ou de solution organique.

Les solutions aqueuses étant commercialement plus facilement disponibles sont utilisées, de préférence.

La concentration de la solution aqueuse de peroxyde d'hydrogène bien que non critique en soi est choisie de façon à introduire le moins d'eau possible dans le milieu réactionnel. On utilise généralement une solution aqueuse de peroxyde d'hydrogène à au moins 20 % en poids de H₂O₂ et, de préférence, aux environs de 70 %.

La quantité de peroxyde d'hydrogène peut aller jusqu'à 1 mole de H₂O₂ pour 1 mole de substrat de formule (I).

Il est cependant préférable pour obtenir un rendement industriellement acceptable d'utiliser un rapport molaire peroxyde d'hydrogène/ substrat de formule (I) de 0,01 à 0,3 et, de préférence, de 0,03 à 0,10.

La quantité d'eau influençant la vitesse de la réaction, il est préférable de minimiser sa présence : l'eau pouvant être apportée dans le milieu réactionnel notamment par les réactifs mis en oeuvre.

Il convient de choisir préférentiellement une teneur initiale du milieu en eau inférieure à 20 % en poids et, de préférence, inférieure à 10 % en poids.

Les teneurs pondérales en eau indiquées sont exprimées par rapport au mélange substrat de formule (I) - peroxyde d'hydrogène - eau.

Cette eau initiale correspond à l'eau introduite avec les réactifs et notamment avec le peroxyde d'hydrogène.

Une variante du procédé de l'invention consiste à ajouter un agent complexant des ions métalliques présents dans le milieu car ceux-ci sont préjudiciables au bon déroulement du procédé de l'invention, notamment dans le cas des phénols où les rendements en produits d'hydroxylation sont faibles. Par conséquent, il est préférable d'inhiber l'action des ions métalliques.

Les ions métalliques néfastes au déroulement de l'hydroxylation sont des ions de métaux de transition et plus particulièrement, les ions fer, cuivre, chrome, cobalt, manganèse et vanadium.

Les ions métalliques sont apportés par les réactifs et notamment les substrats de départ et l'appareillage utilisé. Pour inhiber l'action de ces ions métalliques, il suffit de conduire la réaction en présence d'un ou plusieurs agents complexants stables vis-à-vis du peroxyde d'hydrogène et donnant des complexes ne pouvant être décomposés par les acides forts présents et dans lesquels le métal ne peut plus exercer d'activité chimique.

A titre d'exemples non limitatifs d'agents complexants, on peut faire appel, notamment, aux divers acides phosphoriques tels que, par exemple, l'acide orthophosphorique, l'acide métaphosphorique, l'acide pyrophosphorique, les acides polyphosphoriques, les acides phosphoniques tels que l'acide (1-hydroxyéthylidène)diphosphonique, l'acide phosphonique, l'acide éthylphosphonique, l'acide phénylphosphonique.

On peut également mettre en oeuvre les esters des acides précités et l'on peut mentionner, plus particulièrement, les ortho phosphates de mono- ou di alkyle, de mono- ou dicycloalkyle, de mono- ou dialkylaryle, par exemple, le phosphate d'éthyle ou de diéthyle, le phosphate d'hexyle, le phosphate de cyclohexyle, le phosphate de benzyle.

La quantité d'agent complexant dépend de la teneur du milieu réactionnel en ions métalliques.

Il n'y a évidemment pas de limite supérieure, la quantité d'agents complexants présents pouvant être largement en excès par rapport à celle nécessaire pour complexer les ions métalliques. Généralement, une quantité représentant de 0,01 % et 1 % en poids du milieu réactionnel convient bien.

Conformément au procédé de l'invention, on réalise l'hydroxylation du substrat de formule (I) à une température qui peut être comprise entre 45°C et 140°C.

Une variante préférée du procédé de l'invention consiste à choisir la température entre 60°C et 120°C.

La réaction est conduite avantageusement sous pression atmosphérique.

Des pressions légèrement supérieures ou inférieures peuvent également être utilisées.

On peut aussi conduire le procédé de l'invention sous une atmosphère inerte par exemple sous azote ou bien encore sous argon, l'azote étant préféré notamment compte tenu de son coût réduit.

Le procédé d'hydroxylation est généralement mis en oeuvre sans solvant autre que celui qui provient des réactifs, comme le solvant du peroxyde d'hydrogène.

La réaction peut cependant également être réalisée dans un solvant organique.

Les solvants utilisés doivent êtres stables en présence de peroxyde d'hydrogène.

On peut citer des solvants non polaires comme les hydrocarbures aliphatiques chlorés, par exemple le dichlorométhane, le tétrachlorométhane, le dichloroéthane.

On peut utiliser en particulier dans le cas de l'hydroxylation des éthers de phénols, des solvants plus polaires notamment les éthers, par exemple le sulfolane, le 1,2-diméthoxyéthane mais également l'acétonitrile, le diméthylcarbonate.

D'un point de vue pratique, le procédé selon l'invention est simple à mettre en oeuvre de façon continue ou discontinue.

Le catalyseur mixte de l'invention peut être mis en oeuvre dans le substrat de formule (I) ou dans la solution de peroxyde d'hydrogène.

D'une manière préférée, on choisit l'ordre des réactifs suivants : on introduit le substrat de formule (I), éventuellement l'agent complexant, le mélange d'acides forts.

On porte le milieu réactionnel à la température désirée puis, l'on ajoute la solution de peroxyde d'hydrogène, de manière progressive, par fractions ou en continu.

Selon un mode de réalisation en continu, on peut conduire le procédé de l'invention dans un ou plusieurs réacteurs en cascade.

On introduit dans le premier réacteur, le substrat de formule (I), avec éventuellement l'agent complexant, la solution de peroxyde d'hydrogène ; le mélange d'acides pouvant être introduit seul ou mis en oeuvre dans les autres réactifs.

En fin de réaction, le substrat non transformé, et le cas échéant les acides en excès, sont séparés des produits d'hydroxylation par les moyens usuels, notamment, par distillation et/ou extraction liquide/liquide et sont renvoyés dans la zone réactionnelle.

Dans les exemples, sont mis en oeuvre différents acides dont la force acide est appréciée comparativement à l'acide sulfurique qui est choisi comme référence.

Le ΔpKₐ de l'acide considéré par rapport à l'acide sulfurique est déterminé dans le nitrobenzène par dosage potentiométrique comparatif.

Le protocole de mesure est le suivant :
Chacun des acides (acide protonique fort ou superacide et acide sulfurique) sont dissous à raison de 0,2 mmol dans 50 ml de nitrobenzène et 1 ml d'isopropanol.

Est ajoutée progressivement une solution d'hydroxyde de tétrabutylammonium (solution 0,1 N dans isopropanol).

La courbe de titrage potentiométrique (pH en fonction de volume de solution d'hydroxyde de tétrabutylammonium) est enregistrée à 20°C à l'aide d'électrodes : soit une électrode pH combinée (adaptée au milieu organique) soit une électrode de verre associée à une électrode de référence Ag/AgCl remplie de LiCl à raison de 3 mol/l dans le méthanol.

Les résultats (exprimés en mV ± 0,2) sont donnés en ΔpKₐ par rapport à l'acide sulfurique (1^{ère} acidité) sachant qu'une unité de pkₐ est égale à 60 mV.

On donne quelques exemples de résultats obtenus :
- Acide trifluorométhanesulfonique (triflique) : ΔpKₐ (nitrobenzène) = - 1,0
- HClO₄ : ΔpKₐ (nitrobenzène) =- 0,5
- TFSiH : ΔpKₐ (nitrobenzène): =- 0,1
- Acide p-phénolsulfonique : ΔpKₐ (nitrobenzène) = 0,1

Les exemples qui suivent, illustrent l'invention sans toutefois la limiter.

Dans les exemples, les abréviations suivantes signifient :
Le taux de transformation (TT_{H2O2}) du peroxyde d'hydrogène correspond au rapport entre le nombre de moles de peroxyde d'hydrogène transformées et le nombre de moles de peroxyde d'hydrogène introduites.

Le rendement en diphénols (RR_{diphénols}) correspond au rapport entre le nombre de moles de diphénols formées (pyrocatéchol + hydroquinone) et le nombre de moles de peroxyde d'hydrogène introduites.

Le rendement en pyrocatéchol (RR_{pyrocatèchol}) correspond au rapport entre le nombre de moles de pyrocatéchol formées et le nombre de moles de peroxyde d'hydrogène introduites.

Le rendement en hydroquinone (RR_{hydroquinone}) correspond au rapport entre le nombre de moles d'hydroquinone formées et le nombre de moles de peroxyde d'hydrogène introduites.

La sélectivité en diphénols (RT_{diphénols}) correspond au rapport entre le nombre de moles de diphénols formées (pyrocatéchol + hydroquinone) et le nombre de moles de peroxyde d'hydrogène transformées.

Le ratio PC/HQ est défini par le rapport entre le nombre de moles de pyrocatéchol et le nombre de moles d'hydroquinone.

### EXEMPLES

On donne, ci-après, le protocole opératoire qui va être suivi dans les exemples 1 à 4.

Dans un réacteur en verre de 250 ml muni d'une double enveloppe et équipé d'un système d'agitation type 4 pâles inclinées, d'un réfrigérant ascendant, d'une arrivée d'azote et d'un dispositif de chauffage, on charge à 50°C :
- 117,6 g (1,25 mol) de phénol,
- l'acide pyrophosphorique à raison de 0,03 % du poids du phénol,
- un superacide qui est l'acide perchlorique à 65 % en poids ou l'acide triflique dont les quantités introduites sont mentionnées dans le tableau récapitulatif (I),
- un acide protonique fort qui est soit l'acide sulfurique à 98 % en poids ou de l'acide 4-hydroxybenzènesulfonique (APS) à 65 % en poids, dont les quantités introduites sont mentionnées dans le tableau récapitulatif (I).

Les différents réactifs mis en oeuvre comprennent moins de 1 ppm de métaux notamment de fer.

On porte le mélange à une température de T°C indiquée dans le tableau (I), sous atmosphère d'azote, puis l'on ajoute 3,03 g d'une solution aqueuse de peroxyde d'hydrogène à 70 % en poids (soit 0,0625 mol de peroxyde d'hydrogène), en 15 min, à l'aide d'un pousse seringue.

On observe en général, une augmentation de température accompagnée d'une coloration du mélange réactionnel.

On chauffe ensuite durant 1 h à la température indiquée.

En fin de réaction, on refroidit le mélange réactionnel à 50°C et l'on dose les diphénols formés par chromatographie liquide haute performance.

### Exemples 1 à 3 :

Les conditions et les résultats obtenus sont consignés dans le tableau (I).

**Tableau (I)**

| Référence exemple | 1 | 2 | 3 |
|---|---|---|---|
| Catalyseur | HClO₄/H₂SO₄ | HClO₄/H₂SO₄ | CF₃SO₃H/APS |
| T(°C) | 70°C(15 min) puis 70°C (1 h) | 80°C (15 min) puis 90°C (1 h) | 80°C (15 min) puis 80°C (1 h) |
| HClO₄ % mol / PhOH | 0,0108% | 0,0080 % | - |
| CF₃SO₃H %mol/PhOH | - | - | 0,0042 % |
| H₂SO₄ % mol / PhOH | 0,0597 % | 0,0280 % | - |
| APS % mol / PhOH | - | - | 0,0278 % |
| TT(H₂O₂) | 100 % | 100 % | 100 % |
| RT(HQ)/H₂O₂ | 29 % | 32 % | 25 % |
| RT (PC) / H₂O₂ | 51 % | 51 % | 47 % |
| RT (HQ+PC)/H₂O₂ | 80 % | 83 % | 72 % |
| ratio PC/HQ | 1,72 | 1,58 | 1,93 |

### Exemples comparatifs A et B

Dans ces exemples comparatifs, on met en évidence que la mise en oeuvre d'un couple acide perchlorique/acide pyrophosphorique ne permet pas de jouer sur la sélectivité de la réaction.

On reproduit le protocole opératoire donné au début des exemples à l'exception que l'on ne met pas en oeuvre un acide protonique fort au sens de l'invention.

On ne met en oeuvre dans l'exemple A, que de l'acide perchlorique et dans l'exemple B, un mélange d'acide perchlorique et d'acide pyrophosphorique.

Les conditions et les résultats obtenus sont consignés dans le tableau (II).

**Tableau (II)**

| Référence exemple | A | B |
|---|---|---|
| Catalyseur | Acide perchlorique | Acide perchlorique |
| Agent complexant | | Acide pyrophosphorique |
| T(°C) | 80°C (15 min) puis 90°C (1 h) | 80°C (15 min) puis 90°C (1 h) |
| HClO₄ % mol / PhOH | 0,0330 % | 0,0330 % |
| TT(H₂O₂) | 100 % | 100 % |
| RT (HQ) / H₂O₂ | 35 % | 34 % |
| RT (PC) / H₂O₂ | 48 % | 49 % |
| RT (HQ + PC) / H₂O₂ | 83 % | 83 % |
| ratio PC/HQ | 1,4 | 1,43 |

Il ressort de l'examen de ce tableau que la mise en oeuvre d'un mélange d'acide perchlorique et d'acide pyrophosphorique n'a pas d'influence sur la sélectivité de la réaction puisque le ratio PC/HQ est sensiblement identique.

### Exemple 4

On reproduit le protocole opératoire mais en mettant en oeuvre un mélange d'acide bis-trifluorométhanesulfonimide (TFSiH) et d'APS.

Les conditions et les résultats obtenus sont consignés dans le tableau suivant :

**Tableau (II)**

| Référence exemple | 4 |
|---|---|
| Catalyseur | TFSiH/APS |
| T(°C) | 80°C (15 min) puis 80°C (3 h 30 min) |
| TFSiH % mol / PhOH | 0,0054 % |
| APS % mol / PhOH | 0,0154 % |
| TT(H₂O₂) | 100 % |
| RT(HQ)/H₂O₂ | 28 % |
| RT (PC) / H₂O₂ | 46 % |
| RT (HQ + PC) / H₂O₂ | 74 % |
| ratio PC/HQ | 1,65 |

### Exemple 5

Dans une cascade de trois réacteurs en verre de 500 mL, on introduit en parallèle et en continu le phénol (avec l'agent complexant), le peroxyde d'hydrogène et le catalyseur.

Chaque réacteur à double enveloppe est muni d'un système d'agitation mécanique type 4 pâles inclinées, d'un système de régulation de la température, d'un réfrigérant ascendant et d'une arrivée d'azote.

On charge, à l'aide de pompes, 534 g/h (5,68 mol) de phénol, 15,7 g/h d'une solution aqueuse de peroxyde d'hydrogène à 70 % en poids (soit 0,32 mol/h) et le mélange des acides perchlorique et 4-hydroxybenzène sulfonique [0,18 g/h soit 0,0203 % en moles par rapport au phénol et 0,54 g/h soit 0,0353 % en moles par rapport au phénol respectivement].

Le profil de température est le suivant : 75°C pour le 1^{er} réacteur, 86°C pour le second et 104°C pour le troisième.

Après une durée de stabilisation (environ 1 h), on dose les diphénols formés par chromatographie liquide haute performance et le peroxyde d'hydrogène par potentiométrie.

Les conditions opératoires et résultats obtenus dans le 3^{e} réacteur sont consignés dans le tableau (III).

**Tableau (III)**

| Référence Exemple | 5 |
|---|---|
| Catalyseur APS % mol / PhOH | 0,0353 % |
| Catalyseur HClO₄ % mol / PhOH | 0,0203 % |
| TT H₂O₂ | 91 % |
| RR HQ | 26 % |
| RR PC | 45 % |
| ratio PC/HQ | 1,72 |
| RT(PC +HQ)/H₂O₂ | 78 % |

## Revendications

1. Procédé d'hydroxylation d'un phénol ou d'un éther de phénol présentant au moins un atome d'hydrogène en position ortho et para du groupe hydroxyle ou éther, par réaction dudit phénol ou éther de phénol, avec le peroxyde d'hydrogène, en présence d'un catalyseur acide, **caractérisé par le fait que** la réaction est conduite en présence d'une quantité efficace d'un catalyseur qui est un mélange d'au moins deux acides forts et qui est **caractérisé par le fait que** l'on met en oeuvre un mélange d'au moins deux acides :
- l'un des acides étant choisi parmi les acides protoniques forts, à l'exception des oxacides du phosphore au degré d'oxydation + 5, présentant un pKₐ (S) supérieur ou égal à celui de l'acide sulfurique et un ΔpKₐ (S) par rapport à l'acide sulfurique inférieur ou égal à 4 et supérieur ou égal à 0,
- et l'autre acide étant choisi parmi les superacides.

2. Procédé selon la revendication 1 **caractérisé par le fait que** l'acide protonique fort présente un ΔpKₐ (S) par rapport à l'acide sulfurique inférieur ou égal à 3 et supérieur ou égal à 0.

3. Procédé selon l'une des revendications 1 et 2 **caractérisé par le fait que** l'acide protonique fort est choisi parmi : l'acide sulfurique ; les acides sulfoniques aliphatiques ou aromatiques de préférence l'acide méthanesulfonique, l'acide éthanesulfonique, l'acide benzènesulfonique, les acides toluènesulfoniques, les acides naphtalènesulfoniques ; les acides hydroxybenzènesulfoniques, les acides hydroxybenzoïques sulfonés ; les acides hydroxybenzènedisulfoniques, les acides dihydroxybenzènedisulfoniques, les acides hydroxytoluènesulfoniques, les acides hydroxynaphtalènesulfoniques et les acides hydroxynaphtalènedisulfoniques ; les acides perhalogénoacétiques de préférence l'acide trichloroacétique, l'acide trifluoroacétique et leurs mélanges.

4. Procédé selon la revendication 3 **caractérisé par le fait que** l'acide protonique fort est choisi parmi : l'acide sulfurique ; l'acide 4-hydroxybenzènesulfonique, l'acide 2-hydroxybenzènesulfonique, l'acide 5-sulfosalicylique, l'acide 5,6-dihydroxy-1,3-benzènedisulfonique, l'acide 4,6-dihydroxy-1,3-benzènedisulfonique, l'acide 2,5-dihydroxy-1,4-benzènedisulfonique.

5. Procédé selon l'un des revendications 1 à 4 **caractérisé par le fait que** le superacide présente un pKₐ (S) inférieur à celui de l'acide sulfurique.

6. Procédé selon la revendication 5 **caractérisé par le fait que** le superacide présente un ΔpKₐ par rapport à l'acide sulfurique supérieur ou égal à - 12.

7. Procédé selon l'une des revendications 5 et 6 **caractérisé par le fait que** le superacide présente un ΔpKₐ par rapport à l'acide sulfurique, inférieur ou égal à - 0,1 et de préférence supérieur ou égal à - 8.

8. Procédé selon l'un des revendications 5 à 7 **caractérisé par le fait que** le superacide est choisi parmi : l'acide perchlorique, les acides halogénosulfoniques de préférence l'acide fluorosulfonique, l'acide chlorosulfonique ; les acides perhalogénoalcanesulfonique, de préférence l'acide trifluorométhanesulfonique ; l'acide trifluorométhanesulfinique ; l'acide bis-trifluorométhanesulfonimide et leurs mélanges.

9. Procédé selon la revendication 1 **caractérisé par le fait que** l'on met en oeuvre l'acide perchlorique et l'acide sulfurique ; l'acide perchlorique et l'acide 4-hydroxybenzènesulfonique ; l'acide trifluorométhanesulfonique et l'acide 4-hydroxybenzènesulfonique ; l'acide bis-trifluorométhanesulfonimide et l'acide 4-hydroxybenzènesulfonique.

10. Procédé selon l'une des revendications 1 à 9 **caractérisé par le fait que** le mélange comprend :
- de 60 % à 95 %, de préférence de 80 % à 95 % en moles d'un acide protonique fort,
- de 5 % à 40 % de préférence de 5 % à 20 % en moles d'un superacide.

11. Procédé selon l'une des revendications 1 à 10 **caractérisé par le fait que** la quantité d'acide protonique fort mise en oeuvre exprimée par le rapport entre le nombre de moles dudit acide et le nombre de moles de substrat varie entre 0,01 % et 0,1 %, de préférence entre 0,015 % et 0,06 %.

12. Procédé selon l'une des revendications 1 à 11 **caractérisé par le fait que** la quantité de superacide mise en oeuvre exprimée par le rapport entre le nombre de moles dudit acide et le nombre de moles de substrat varie entre 0,002 % et 0,05 %, de préférence entre 0,003 % et 0,03 %.

13. Procédé selon l'une des revendications 1 à 12 **caractérisé par le fait que** le substrat répond à la formule générale (I) : 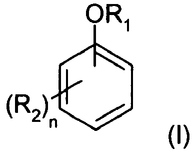 dans ladite formule :
- n est un nombre de 0 à 4, de préférence égal à 0, 1 ou 2,
- R₁ représente un atome d'hydrogène, un groupe alkyle, cycloalkyle, aryle, aralkyle,
- R₂, identiques ou différents, représentent un groupe alkyle, un groupe alkoxy, un groupe hydroxyle, un atome d'halogène, un groupe halogéno- ou perhalogénoalkyle.

14. Procédé selon la revendication 13 **caractérisé par le fait que** le substrat est choisi parmi : le phénol ; les éthers aliphatiques de phénols ; les monoalkylphénols, les dialkylphénols, les trialkylphénols avec des groupes alkyle en C₁-C₄ ; les alkoxyphénols avec des groupes alkoxy en C₁-C₄.

15. Procédé selon la revendication 13 **caractérisé par le fait que** le substrat est choisi parmi : le phénol ; l'anisole, le phénétole ; l'o-crésol, le m-crésol ; le 2-méthoxyphénol (le gaïacol), le 2-éthoxyphénol (le guétol).

16. Procédé selon l'une des revendications 1 à 15 **caractérisé par le fait que** le rapport molaire peroxyde d'hydrogène/ substrat de formule (I) varie de 0,01 à 0,3 et, de préférence, de 0,03 à 0,10.

17. Procédé selon l'une des revendications 1 à 16 **caractérisé par le fait que** l'on opère en présence d'un agent complexant des ions de métaux de transition, stables dans les conditions de réaction de préférence les acides phosphoriques et préférentiellement l'acide orthophosphorique, l'acide métaphosphorique, l'acide pyrophosphorique ; les acides polyphosphoriques ; les acides phosphoniques de préférence l'acide (1-hydroxyéthylidène)diphosphonique, l'acide phosphonique, l'acide éthylphosphonique, l'acide phénylphosphonique et leurs esters-acides.

18. Procédé selon l'une des revendications 1 à 17 **caractérisé par le fait que** l'on opère à une température comprise entre 45°C et 140°C, de préférence, entre 60°C et 120°C.

19. Procédé selon l'une des revendications 1 à 18 **caractérisé par le fait que** l'on introduit le substrat de formule (I), éventuellement l'agent complexant, le mélange d'acides forts, l'on porte le milieu réactionnel à la température désirée puis, l'on ajoute la solution de peroxyde d'hydrogène, de manière progressive, par fractions ou en continu.

20. Procédé selon l'une des revendications 1 à 19 **caractérisé par le fait qu'**il est mis en oeuvre en discontinu ou en continu.

## Patentansprüche

1. Verfahren zur Hydroxylierung eines Phenols oder eines Phenolethers mit mindestens einem Wasserstoffatom in ortho- und para-Stellung der Hydroxyl- bzw. Ethergruppe durch Umsetzung des Phenols oder Phenolethers mit Wasserstoffperoxid in Gegenwart eines sauren Katalysators, **dadurch gekennzeichnet, dass** die Umsetzung in Gegenwart einer wirksamen Menge eines Katalysators durchgeführt wird, bei dem es sich um eine Mischung von mindestens zwei starken Säuren handelt, und der **dadurch gekennzeichnet ist, dass** eine Mischung von mindestens zwei Säuren verwendet wird:
- wobei eine der Säuren aus starken Protonensäuren, ausgenommen Oxosäuren von Phosphor in der Oxidationsstufe +5, mit einem pKₐ (S), der größer oder gleich dem pKₐ (S) von Schwefelsäure ist, und einem ΔpKₐ (S) in Bezug auf Schwefelsäure kleiner gleich 4 und größer gleich 0 ausgewählt wird
- und die andere Säure aus Supersäuren ausgewählt wird.

2. Verfahren nach Anspruch 1, **dadurch gekennzeichnet, dass** die starke Protonensäure einen ΔpKₐ (S) in Bezug auf Schwefelsäure kleiner gleich 3 und größer gleich 0 aufweist.

3. Verfahren nach Anspruch 1 oder 2, **dadurch gekennzeichnet, dass** die starke Protonensäure aus Schwefelsäure; aliphatischen oder aromatischen Sulfonsäuren, vorzugsweise Methansulfonsäure, Ethansulfonsäure, Benzolsulfonsäure, Toluolsulfonsäuren, Naphthalinsulfonsäuren; Hydroxybenzolsulfonsäuren, sulfonierten Hydroxybenzolsäuren; Hydroxybenzoldisulfonsäuren, Dihydroxybenzoldisulfonsäuren, Hydroxytoluolsulfonsäuren, Hydroxynaphthalinsulfonsäuren und Hydroxynaphthalindisulfonsäuren; Perhalogenessigsäuren, vorzugsweise Trichloressigsäure, Trifluoressigsäure, und Mischungen davon ausgewählt wird.

4. Verfahren nach Anspruch 3, **dadurch gekennzeichnet, dass** die starke Protonensäure aus Schwefelsäure; 4-Hydroxybenzolsulfonsäure, 2-Hydroxybenzolsulfonsäure, 5-Sulfosalicylsäure, 5,6-Dihydroxy-1,3-benzoldisulfonsäure, 4,6-Dihydroxy-1,3-benzol-disulfonsäure und 2,5-Dihydroxy-1,4-benzol-disulfonsäure ausgewählt wird.

5. Verfahren nach einem der Ansprüche 1 bis 4, **dadurch gekennzeichnet, dass** die Supersäure einen pKₐ (S) aufweist, der kleiner als der pKₐ (S) von Schwefelsäure ist.

6. Verfahren nach Anspruch 5, **dadurch gekennzeichnet, dass** die Supersäure einen ΔpKₐ (S) in Bezug auf Schwefelsäure größer gleich -12 aufweist.

7. Verfahren nach Anspruch 5 oder 6, **dadurch gekennzeichnet, dass** die Supersäure einen ΔpKₐ in Bezug auf Schwefelsäure kleiner gleich -0,1 und vorzugsweise größer gleich -8 aufweist.

8. Verfahren nach einem der Ansprüche 5 bis 7, **dadurch gekennzeichnet, dass** die Supersäure aus Perchlorsäure, Halogensulfonsäuren, vorzugsweise Fluorssulfonsäure, Chlorsulfonsäure; Perhalogenalkansulfonsäuren, vorzugsweise Trifluormethansulfonsäure; Trifluormethansulfinsäure; Bistrifluormethansulfonimidsäure und Mischungen davon ausgewählt wird.

9. Verfahren nach Anspruch 1, **dadurch gekennzeichnet, dass** man Perchlorsäure und Schwefelsäure; Perchlorsäure und 4-Hydroxybenzolsulfonsäure; Trifluormethansulfonsäure und 4-Hydroxybenzolsulfonsäure; Bistrifluormethansulfonimidsäure und 4-Hydroxybenzolsulfonsäure verwendet.

10. Verfahren nach einem der Ansprüche 1 bis 9, **dadurch gekennzeichnet, dass** die Mischung Folgendes umfasst:
- 60 bis 95 Mol-% und vorzugsweise 80 bis 95 Mol-% einer starken Protonensäure,
- 5 bis 40 Mol-% und vorzugsweise 5 bis 20 Mol-% einer Supersäure.

11. Verfahren nach einem der Ansprüche 1 bis 10, **dadurch gekennzeichnet, dass** die verwendete Menge von starker Protonensäure, ausgedrückt durch das Verhältnis zwischen der Zahl der Mole der Säure und der Zahl der Mole des Substrats zwischen 0,01% und 0,1% und vorzugsweise zwischen 0,015% und 0,06% variiert.

12. Verfahren nach einem der Ansprüche 1 bis 11, **dadurch gekennzeichnet, dass** die verwendete Menge von Supersäure, ausgedrückt durch das Verhältnis zwischen der Zahl der Mole der Säure und der Zahl der Mole des Substrats zwischen 0,002% und 0,05% und vorzugsweise zwischen 0,003% und 0,03% variiert.

13. Verfahren nach einem der Ansprüche 1 bis 12, **dadurch gekennzeichnet, dass** das Substrat der allgemeinen Formel (I) entspricht: 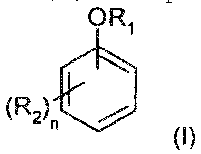 wobei in der Formel:
- n für eine Zahl von 0 bis 4 steht und vorzugsweise gleich 0, 1 oder 2 ist;
- R₁ für ein Wasserstoffatom oder eine Alkyl-, Cycloalkyl-, Aryl- oder Aralkylgruppe steht und
- die Gruppen R₂ gleich oder verschieden sind und für eine Alkylgruppe, eine Alkoxygruppe, eine Hydroxylgruppe, ein Halogenatom oder eine Halogenalkyl- oder Perhalogenalkylgruppe stehen.

14. Verfahren nach Anspruch 13, **dadurch gekennzeichnet, dass** das Substrat aus Phenol; aliphatischen Ethern von Phenolen; Monoalkylphenolen, Dialkylphenolen, Trialkylphenolen mit C₁-C₄-Alkylgruppen und Alkoxyphenolen mit C₁-C₄-Alkoxygruppen ausgewählt wird.

15. Verfahren nach Anspruch 13, **dadurch gekennzeichnet, dass** das Substrat aus Phenol; Anisol, Phenetol; o-Kresol, m-Kresol; 2-Methoxyphenol (Guajacol) und 2-Ethoxyphenol (Guaethol) ausgewählt wird.

16. Verfahren nach einem der Ansprüche 1 bis 15, **dadurch gekennzeichnet, dass** das Molverhältnis von Wasserstoffperoxid zu Substrat der Formel (I) von 0,01 bis 0,3 und vorzugsweise von 0,03 bis 0,10 variiert.

17. Verfahren nach einem der Ansprüche 1 bis 16, **dadurch gekennzeichnet, dass** man in Gegenwart von einem Komplexbildner für Übergangsmetallionen, der unter den Reaktionsbedingungen stabil ist, vorzugsweise Phosphorsäuren und bevorzugt Orthophosphorsäure, Metaphosphorsäure, Pyrophosphorsäure; Polyphosphorsäuren; Phosphonsäuren, vorzugsweise 1-(Hydroxyethyliden)diphosphonsäure, Phosphonsäure, Ethylphosphonsäure, Phenylphosphonsäure und Estersäuren davon, arbeitet.

18. Verfahren nach einem der Ansprüche 1 bis 17, **dadurch gekennzeichnet, dass** man bei einer Temperatur zwischen 45°C und 140°C und vorzugsweise zwischen 60°C und 120°C arbeitet.

19. Verfahren nach einem der Ansprüche 1 bis 18, **dadurch gekennzeichnet, dass** man das Substrat der Formel (I), gegebenenfalls den Komplexbildner und die Mischung von starken Säuren einträgt, dass Reaktionsmedium auf die gewünschte Temperatur erhitzt und dann die Lösung von Wasserstoffperoxid allmählich, portionsweise oder kontinuierlich zugibt.

20. Verfahren nach einem der Ansprüche 1 bis 19, **dadurch gekennzeichnet, dass** es diskontinuierlich oder kontinuierlich durchgeführt wird.

## Claims

1. Process for the hydroxylation of a phenol or of a phenol ether having at least one hydrogen atom at the ortho and the para position of the hydroxyl or ether group, by reaction of said phenol or phenol ether, with hydrogen peroxide, in the presence of an acid catalyst, **characterized in that** the reaction is carried out in the presence of an effective amount of a catalyst which is a mixture of at least two strong acids and which is **characterized in that** a mixture of at least two acids is used:
- one of the acids being selected from strong protonic acids, with the exception of the oxyacids of phosphorus in the +5 oxidation state, having a pKₐ (S) greater than or equal to that of sulphuric acid and a ΔpKₐ (S) with respect to sulphuric acid less than or equal to 4 and greater than or equal to 0,
- and the other acid being selected from superacids.

2. Process according to Claim 1, **characterized in that** the strong protonic acid has a ΔpKₐ (S) with respect to sulphuric acid less than or equal to 3 and greater than or equal to 0.

3. Process according to either of Claims 1 and 2, **characterized in that** the strong protonic acid is selected from: sulphuric acid; aliphatic or aromatic sulphonic acids, preferably methanesulphonic acid, ethanesulphonic acid, benzenesulphonic acid, toluenesulphonic acids, naphthalenesulphonic acids; hydroxybenzenesulphonic acids, sulphonated hydroxybenzoic acids; hydroxybenzenedisulphonic acids, dihydroxybenzenedisulphonic acids, hydroxytoluenesulphonic acids, hydroxynaphthalenesulphonic acids and hydroxynaphthalenedisulphonic acids; perhaloacetic acids, preferably trichloroacetic acid, trifluoroacetic acid and mixtures thereof.

4. Process according to Claim 3, **characterized in that** the strong protonic acid is selected from: sulphuric acid; 4-hydroxybenzenesulphonic acid, 2-hydroxybenzenesulphonic acid, 5-sulphosalicylic acid, 5,6-dihydroxy-1,3-benzenedisulphonic acid, 4,6-dihydroxy-1,3-benzenedisulphonic acid and 2,5-dihydroxy-1,4-benzenedisulphonic acid.

5. Process according to one of Claims 1 to 4, **characterized in that** the superacid has a pKₐ(S) below that of sulphuric acid.

6. Process according to Claim 5, **characterized in that** the superacid has a ΔpKₐ relative to sulphuric acid greater than or equal to -12.

7. Process according to either of Claims 5 and 6, **characterized in that** the superacid has a ΔpKₐ relative to sulphuric acid less than or equal to -0.1 and preferably greater than or equal to -8.

8. Process according to one of Claims 5 to 7, **characterized in that** the superacid is selected from: perchloric acid, halosulphonic acids, preferably fluorosulphonic acid or chlorosulphonic acid; perhaloalkanesulphonic acids, preferably trifluoromethanesulphonic acid; trifluoromethanesulphinic acid; bis(trifluoromethanesulphonyl)imide acid and mixtures thereof.

9. Process according to Claim 1, **characterized in that** use is made of perchloric acid and sulphuric acid; perchloric acid and 4-hydroxybenzenesulphonic acid; trifluoromethanesulphonic acid and 4-hydroxybenzenesulphonic acid; bis(trifluoromethanesulphonyl)imide acid and 4-hydroxybenzenesulphonic acid.

10. Process according to one of Claims 1 to 9, **characterized in that** the mixture comprises:
- from 60 mol% to 95 mol%, preferably from 80 mol% to 95 mol% of a strong protonic acid; and
- from 5 mol% to 40 mol%, preferably from 5 mol% to 20 mol% of a superacid.

11. Process according to one of Claims 1 to 10, **characterized in that** the amount of strong protonic acid used expressed by the ratio between the number of moles of said acid and the number of moles of substrate varies between 0.01% and 0.1%, preferably between 0.015% and 0.06%.

12. Process according to one of Claims 1 to 11, **characterized in that** the amount of superacid used expressed by the ratio between the number of moles of said acid and the number of moles of substrate varies between 0.002% and 0.05%, preferably between 0.003% and 0.03%.

13. Process according to one of Claims 1 to 12, **characterized in that** the substrate corresponds to the general formula (I) : 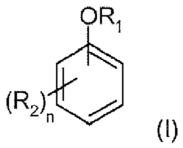 in said formula:
- n is a number from 0 to 4, preferably equal to 0, 1 or 2;
- R₁ represents a hydrogen atom, or an alkyl, cycloalkyl, aryl or aralkyl group; and
- R₂, which are identical or different, represent an alkyl group, an alkoxy group, a hydroxyl group, a halogen atom, a haloalkyl or perhaloalkyl group.

14. Process according to Claim 13, **characterized in that** the substrate is selected from: phenol; aliphatic ethers of phenols; monoalkylphenols, dialkylphenols, trialkylphenols with C₁-C₄ alkyl groups; and alkoxyphenols with C₁-C₄ alkoxy groups.

15. Process according to Claim 13, **characterized in that** the substrate is selected from: phenol, anisole, phenetole; o-cresol, m-cresol; 2-methoxyphenol (guaiacol), and 2-ethoxyphenol (guaethol).

16. Process according to one of Claims 1 to 15, **characterized in that** the hydrogen peroxide/substrate of formula (I) molar ratio varies from 0.01 to 0.3 and, preferably, from 0.03 to 0.10.

17. Process according to one of Claims 1 to 16, **characterized in that** it is carried out in the presence of an agent for complexing transition metal ions, which is stable under the reaction conditions, preferably phosphoric acids and preferentially orthophosphoric acid, metaphosphoric acid, pyrophosphoric acid; polyphosphoric acids; phosphonic acids, preferably (1-hydroxyethylidene)diphosphonic acid, phosphonic acid, ethylphosphonic acid, phenylphosphonic acid and their ester acids.

18. Process according to one of Claims 1 to 17, **characterized in that** it is carried out at a temperature between 45°C and 140°C, preferably between 60°C and 120°C.

19. Process according to one of Claims 1 to 18, **characterized in that** the substrate of formula (I), optionally the complexing agent and the mixture of strong acids are introduced, the reaction medium is brought to the desired temperature and then the solution of hydrogen peroxide is added, gradually, in fractions or continuously.

20. Process according to one of Claims 1 to 19, **characterized in that** it is carried out in batch mode or in continuous mode.
